# EUROPEAN PATENT SPECIFICATION

(11) **EP 1 743 568 B1**
(45) Date of publication and mention of the grant of the patent: **26.08.2015**
(21) Application number: 06014583.6
(22) Date of filing: 13.07.2006
(51) Int. Cl.: A61B 1/00, A61B 1/06, A61B 1/045, A61B 1/05, A61B 1/04, A61B 5/00

(54) **Image processing device**
Bildverarbeitungsvorrichtung
Dispositif de traitement de l'image

(30) Priority: 13.07.2005 JP 2005204753
(43) Date of publication of application: 17.01.2007
(73) Proprietor: OLYMPUS MEDICAL SYSTEMS CORP., Tokyo (JP)
(72) Inventor: Imaizumi, Katsuichi, c/o Olympus Intellectual, Hachioji-shi, Tokyo 192-8512 (JP); Takasugi, Kei, c/o Olympus Intellectual, Hachioji-shi, Tokyo 192-8512 (JP); Okusho, Susumu, c/o Olympus Intellectual, Hachioji-shi, Tokyo 192-8512 (JP)
(74) Representative: von Hellfeld, Axel

(56) References cited:
- EP-A- 1 484 001
- JP-A- 2001 109 445
- US-A1- 4 712 133
- US-A1- 5 034 888
- US-A1- 5 387 928
- US-A1- 5 772 580

## Description

### BACKGROUND OF THE INVENTION

### 1. Field of the Invention

The present invention relates to an image processing device which enables observation using multiple types of observation light.

### 2. Description of the Related Art

Currently, electronic endoscopes are widely employed in medical institutions. With this electronic endoscope, the gastrointestinal tract such as the esophagus, stomach, small intestine, large intestine or the like, or the trachea such as the lungs or the like, can be observed by inserting the insertion portion of the scope into a body cavity, and also various types of treatment processing can be performed using a treatment tool which is to be inserted into a treatment tool channel as necessary.

Particularly, in Japan, surface sequential type endoscope devices are widely employed. This surface sequential type endoscope device sequentially irradiates light such as red, green, blue, or the like upon a subject by passing the light through an optical filter from a light source device, or the like, and receives this at a monochrome image capturing device, and subjects this to signal processing within a processor, and then outputs this to a display device as a color image.

The above processor includes color enhancement processing, which is performed to facilitate detection of a lesion, as internal signal processing. This color enhancement processing is processing for clearly distinguishing a normal mucous membrane and a diseased mucous membrane based on the difference between the colors of the two by enhancing a color including a lot of hemoglobin on the basis of the amount of hemoglobin included in a living body mucous membrane.

Also, diagnosis using an endoscope is generally diagnosis using ordinary light observation, but in recent years, diagnosis using autologous fluorescent observation has been performed. Here, this diagnosis using ordinary light observation is diagnosis for displaying the same color image as that can be viewed with the naked eyes, and observing this. Also, the above diagnosis using autologous fluorescent observation is diagnosis for displaying the image based on the autologous fluorescence which employed the autologous fluorescence of an organic tissue, and observing this. Specifically, with the diagnosis using autologous fluorescent observation, diagnosis is performed by utilizing that when irradiating excitation light from ultraviolet through blue upon an organic tissue, and the autologous fluorescent spectrums to be output from the organic tissue differ between a normal mucous membrane and a tumor.

The autologous fluorescent image to be employed for the above diagnosis using autologous fluorescent light observation is displayed on a monitor along with a reflection light image to be reflected at the living body and returned. The autologous fluorescent image is an image which can be clearly recognized as a color difference between a diseased portion and a normal portion by displaying the images corresponding to the autologous fluorescent spectrums on the monitor while assigning a different color to each thereof. Fluorescence is weak, so a great amount of noise is included in a fluorescent image, and accordingly, it is often the case that a fluorescent observation processor is provided with a noise removal circuit.

Also, for example, as disclosed in Japanese Unexamined Patent Application Publication No. 2002-95635, a device has been proposed for irradiating light having a narrower band than that of ordinary observation light employed for normal light observation upon organic tissue, and observing this using an image called a narrowband light observation image (NBI: Narrow Band Imaging). The narrowband light observation image is an image for enabling the blood vessel of a mucous membrane surface to be observed with excellent contrast.

The observation using this narrowband light observation image is performed using narrowband light, so an image having a color tone different from an endoscope image to be employed for ordinary light observation is displayed. Accordingly, a narrowband light observation processor performs color adjustment by providing a color conversion circuit inside thereof, converts the narrowband light observation image into a color tone suitable for determining a lesion, and then outputs this to a monitor for display.

Also, for example, Japanese Unexamined Patent Application Publication No. 2004-166913 and so forth have disclosed a contact-type observation endoscope device which enables observation to be performed locally with high magnification by contacting the tip of the endoscope to an observation portion.

The ordinary light observation, autologous fluorescent observation, narrowband light observation, and contact-type observation can be integrated into one system by employing an illumination device capable of switching over illumination light.

The autologous fluorescent observation is an observation method for detecting a diseased portion principally, so it is effective to observe a diseased portion from a distant view to take a wide view. Inversely, the narrowband light observation enables detailed structures to be observed, and so is employed for observing the detected lesion further in detail, and makes a significant contribution when approaching and enlarging a subject. Also, with the contact-type observation, the best image can be obtained in a state in which the scope tip end portion is in contact with a subject.

However, with the observation modes of the ordinary observation, fluorescent observation, narrowband light observation, contact-type observation, and so forth, a favorable observation distance differs depending on the observation mode to be employed. Accordingly, when displaying two images having a different observation mode on the two screens of a monitor or the like, displaying the two screens with moving images leads to a problem wherein the image of one observation mode becomes an image not suitable for observation.

EP 1 484 001 discloses a video processor comprising image processing means including a free switch to perform freezing of images of different observation modes.

### SUMMARY OF THE INVENTION

Accordingly, it is an object of the present invention to provide an image processing device which enables the images displayed on the two screens on the same monitor having a different observation mode to be converted into images suitable for observation.

To this end, an image processing device according to claim 1 is provided.

Other features and profits of the present invention will be apparent sufficiently in the following description.

### BRIEF DESCRIPTION OF THE DRAWINGS

Fig. 1 through Fig. 8 relate to a first embodiment of the present invention.
Fig. 1 is a configuration diagram illustrating the configuration of an endoscope device;
Fig. 2 is a diagram illustrating the configuration of the rotary filter in Fig. 1;
Fig. 3 is a block diagram illustrating the configuration of the freeze memory unit in Fig. 1;
Fig. 4 is a first diagram describing the actions of the endoscope device in Fig. 1;
Fig. 5 is a second diagram describing the actions of the endoscope device in Fig. 1;
Fig. 6 is a third diagram describing the actions of the endoscope device in Fig. 1;
Fig. 7 is a fourth diagram describing the actions of the endoscope device in Fig. 1;
Fig. 8 is a fifth diagram describing the actions of the endoscope device in Fig. 1.
Fig. 9 through Fig. 11 relate to a second embodiment of the present invention.

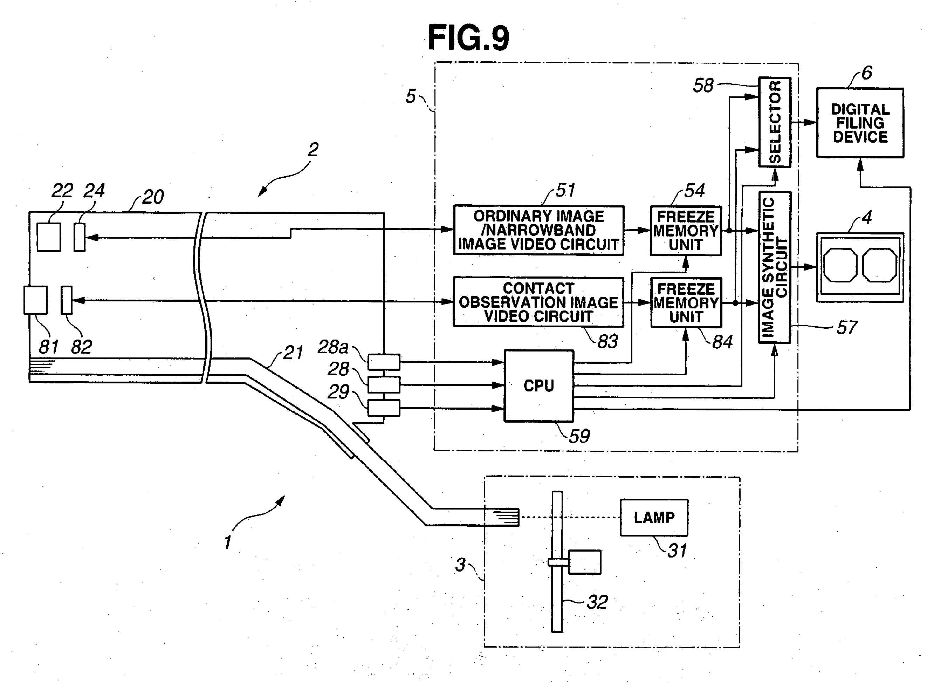

Fig. 9 is a configuration diagram illustrating the configuration of an endoscope device;

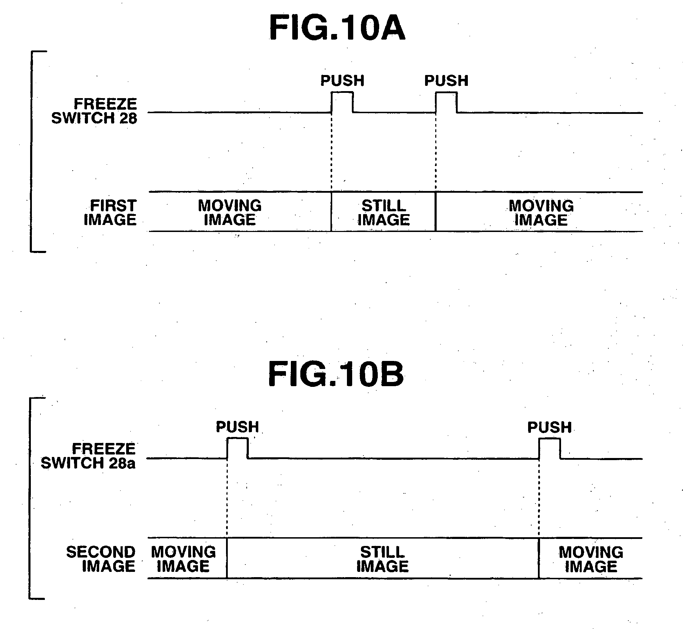

Fig. 10A is a first diagram describing the actions of the endoscope device in Fig. 9;
Fig. 10B is a second diagram describing the actions of the endoscope device in Fig. 9;

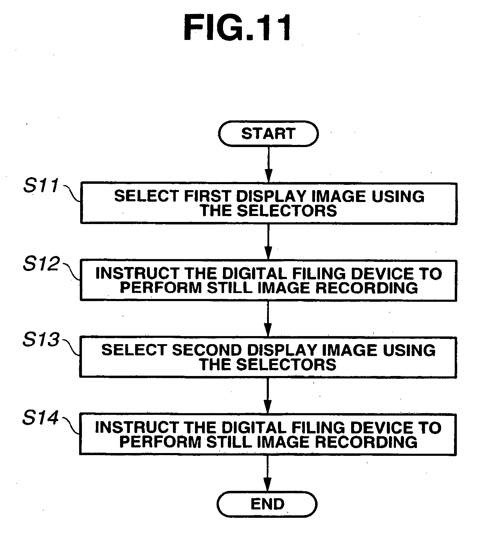

Fig. 11 is a third diagram describing the actions of the endoscope device in Fig. 9.

### DETAILED DESCRIPTION OF THE PREFERRED EMBODIMENTS

### First Embodiment

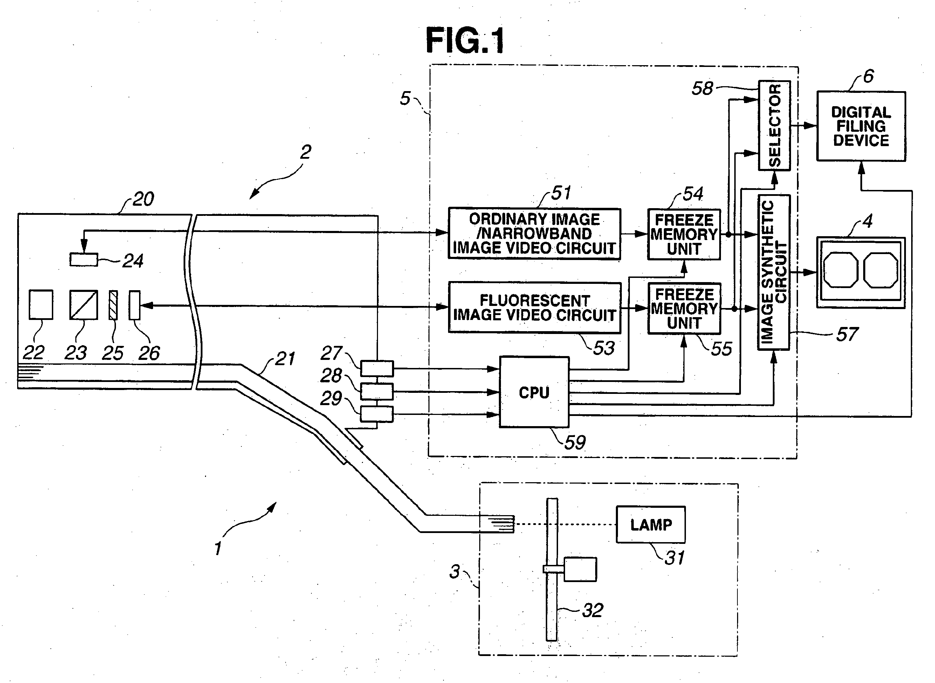

As illustrated in Fig. 1, an endoscope device 1 according to the present embodiment comprises an electronic endoscope 2, a light source device 3, a video processor 5, and a digital filing device 6.

The electronic endoscope 2 is an electronic endoscope capable of observing a subject within a body cavity using a plurality of observation light. The light source device 3 is a light source device for supplying a plurality of observation light to the electronic endoscope 2. The video processor 5 is a video processor for subjecting the image capturing signal of the subject captured with a plurality of observation light by the electronic endoscope 2 to signal processing, and displaying the image of the subject on a monitor 4. The digital filing device 6 is a digital filing device for recording the image of the subject generated by the video processor 5.

The light source device 3 comprises a lamp 31 serving as a xenon light source for emitting white light for example, and a rotary filter 32 for converting the white light into a plurality of observation light, and supplying these to a light guide fiber 21 inserting through the inside of a flexible insertion portion 20 of the electronic endoscope 2.

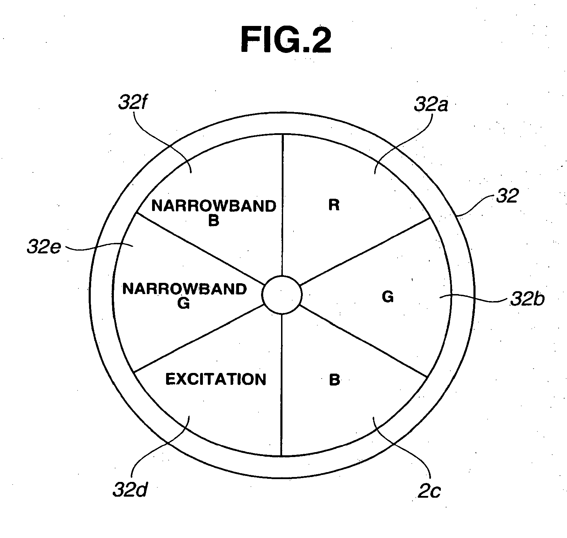

As illustrated in Fig. 2, the rotary filter 32 comprises a R (red) filter 32a, a G (green) filter 32b, and a B (blue) filter 32c, which convert white light into RGB light serving as ordinary observation light, an extinction light filter 32d for converting white light into ultraviolet through blue excitation light, a narrowband G filter 32e for converting white light into narrowband G light which is narrower than the transmission band of the G (green) filter, and a narrowband B filter 32f for converting white light into B light which is narrower than the transmission band of the B (blue) filter, and is configured so as to convert white light into a plurality of surface sequential observation light by rotating the rotary filter 32.

Now, returning to Fig. 1, the electronic endoscope 2 comprises an objective lens 22, a beam splitter 23, ordinary observation light/narrowband light CCD 24, a fluorescent CCD 26, a freeze selection switch 27, a freeze switch 28, and a release switch 29. The freeze selection switch 27, freeze switch 28, and release switch 29 are provided in an operating unit at the base end side of the insertion portion 20.

The objective lens 22 is an objective lens for receiving the optical image of the subject illuminated by a plurality of observation light which transmitted through the light guide fiber 21.

The beam splitter 23 is an optical element for separating the optical image of the subject received from the objective lens 22 into two directions.

The ordinary observation light/narrowband light CCD 24 is a CCD for capturing the optical image of the subject of one of ordinary observation light and narrowband light separated by the beam splitter 23.

The fluorescent CCD 26 is a CCD for capturing the optical image of the subject of autologous fluorescence excited by the other excitation light separated by the beam splitter 23 via an excitation light cut filter 25.

The freeze selection switch 27 is a switch for selecting the freeze processing of the image captured by the ordinary observation light/narrowband light CCD 24, or the image captured by the fluorescent CCD 26.

The freeze switch 28 is a switch serving as freeze instructing means for executing the freeze processing of the image selected by the freeze selection switch 27.

The release switch 29 is a switch for instructing the digital filing device 6 to perform image recording.

The video processor 5 comprises an ordinary image/narrowband image video circuit 51, a fluorescent image video circuit 53, an image synthetic circuit 57, a selector 58, and a CPU 59.

The ordinary image/narrowband image video circuit 51 is a circuit for subjecting the image capturing signal captured by the ordinary observation light/narrowband light CCD 24 to signal processing to generate an ordinary observation light image or narrowband light image.

The fluorescent image video circuit 53 is a circuit for subjecting the image capturing signal using the autologous fluorescence captured by the fluorescent CCD 26 to signal processing to generate a fluorescent image.

The image synthetic circuit 57 is a circuit serving as image synthesizing means for synthesizing the images generated by the ordinary image/narrowband image video circuit 51 and the fluorescent image video circuit 53 via freeze memory units 54 and 55 to output this to the monitor 4.

The selector 58 is a selector for selectively outputting the images generated by the ordinary image/narrowband image video circuit 51 and the fluorescent image video circuit 53 via the freeze memory units 54 and 55 to the digital filing device 6.

The CPU 59 is a control circuit serving as processing control means for controlling the freeze memory units 54 and 55, image synthetic circuit 57, and selector 58 depending on the freeze selection switch 27, freeze switch 28, and release switch 29 of the electronic endoscope 3.

Here, the ordinary image/narrowband image video circuit 51 and the fluorescent image video circuit 53 are configured as a plurality of observation mode image processing means.

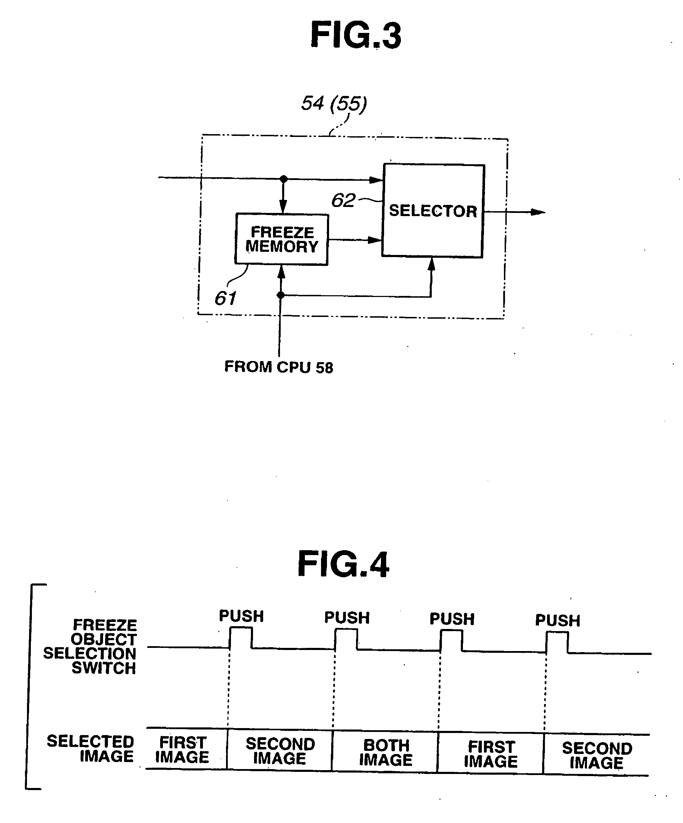

The freeze memory units 54 and 55 have the same configuration, for example, as illustrated in Fig. 3, the freeze memory 54 comprises freeze memory 61 for freezing the image generated by the ordinary image/narrowband image video circuit 51 for the worth of one screen, and storing this, and a selector 62 for selectively outputting the image generated by the ordinary image/narrowband image video circuit 51 and the still image stored in the freeze memory 61 to the image synthetic circuit 57. The freeze memory 61 and the selector 62 are configured so as to be controlled by the CPU 59.

Description will be made regarding the actions according to the present embodiment thus configured. The insertion portion 20 of the electronic endoscope 3 is inserted into the body, and a plurality of observation light (ordinary observation light, narrowband light, and excitation light) are sequentially supplied from the light source device 3 to irradiate this upon the subject.

The electronic endoscope 3 captures the optical image of the subject using ordinary observation light and narrowband light by the ordinary observation light/narrowband light CCD 24, or captures the optical image of the autologous fluorescence using excitation light by the fluorescent CCD 26.

Subsequently, the video processor 5 subjects the image capturing signal of the ordinary observation light/narrowband light CCD 24 to signal processing using ordinary observation light at the ordinary image video circuit 51 to generate an ordinary light image or narrowband light image, and also subjects the image capturing signal of the fluorescent CCD 26 using autologous fluorescence to signal processing at the fluorescent image video circuit 53 to generate a fluorescent image.

As illustrated in Fig. 4, whenever the freeze selection switch 27 is operated, the CPU 59 sets a freeze processing object image in the cycle of a first image (e.g., the output image of the ordinary image/narrowband image video circuit 51), a second image (e.g., the output image of the fluorescent image video circuit 53), and both images (the output image of the ordinary image/narrowband image video circuit 51 and the output image of the fluorescent image video circuit 53).

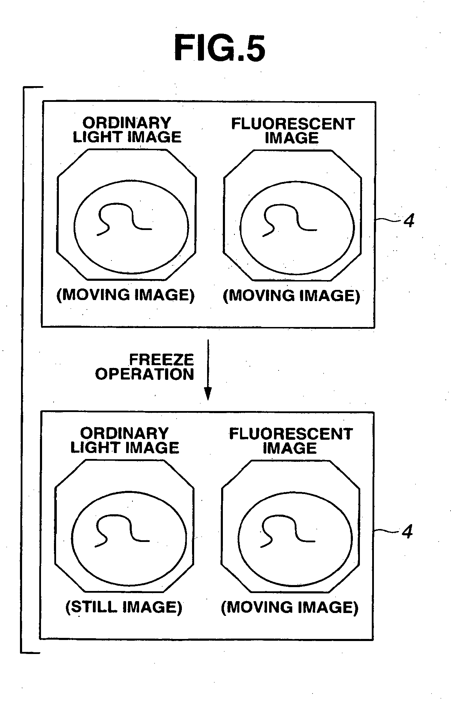

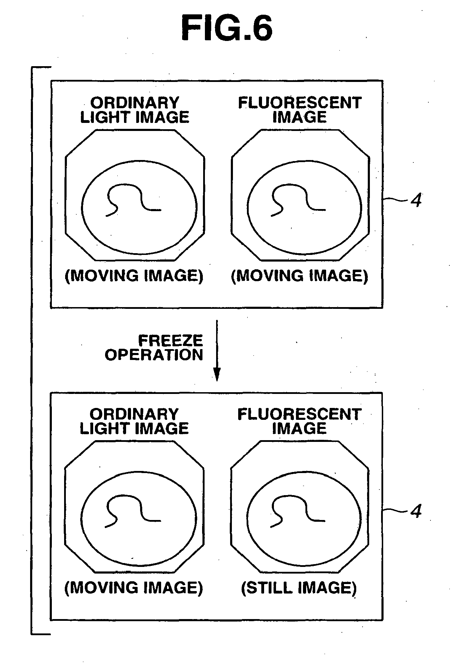

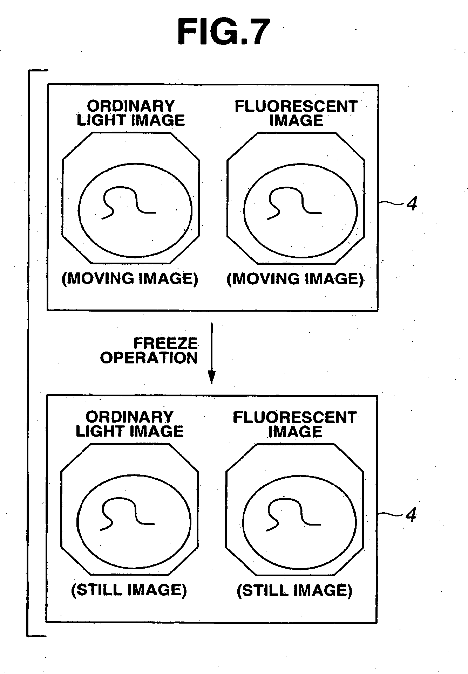

Thus, in a state in which a freeze processing object image has been set, upon detecting operation of the freeze switch 28, the CPU 59 controls the freeze memory units 54 and 55, and the image synthetic circuit 57 to selectively set the images of the two screens to be displayed on the monitor 4 as a still image. That is to say, for example,
(1) As illustrated in Fig. 5, in the event that the two screen images of the monitor 4 are the moving images of an ordinary light observation image or narrowband light image, and a fluorescent image, the freeze selection switch 27 is operated, and the CPU 59 sets the ordinary light observation image or narrowband light image (first image) to a freeze processing object image, upon the CPU 59 detecting operation of the freeze switch 28, the video processor 5 displays the fluorescent image on the monitor 4 as a moving image, and displays the ordinary light observation image or narrowband light image alone on the monitor 4 as a still image.
(2) Also, as illustrated in Fig. 6, in the event that the two screen images of the monitor 4 are the moving images of an ordinary light observation image or narrowband light image, and a fluorescent image, the freeze selection switch 27 is operated, and the CPU 59 sets the fluorescent image (second image) to a freeze processing object image, upon the CPU 59 detecting operation of the freeze switch 28, the video processor 5 displays the ordinary light observation image or narrowband light image on the monitor 4 as a moving image, and displays the fluorescent image alone on the monitor 4 as a still image.
(3) Further, as illustrated in Fig. 7, in the event that the two screen images of the monitor 4 are the moving images of an ordinary light observation image or narrowband light image, and a fluorescent image, the freeze selection switch 27 is operated, and the CPU 59 sets both images (first and second images) to freeze processing object images, upon the CPU 59 detecting operation of the freeze switch 28, the video processor 5 displays the ordinary light observation image or narrowband light image, and the fluorescent image on the monitor 4 as still images.

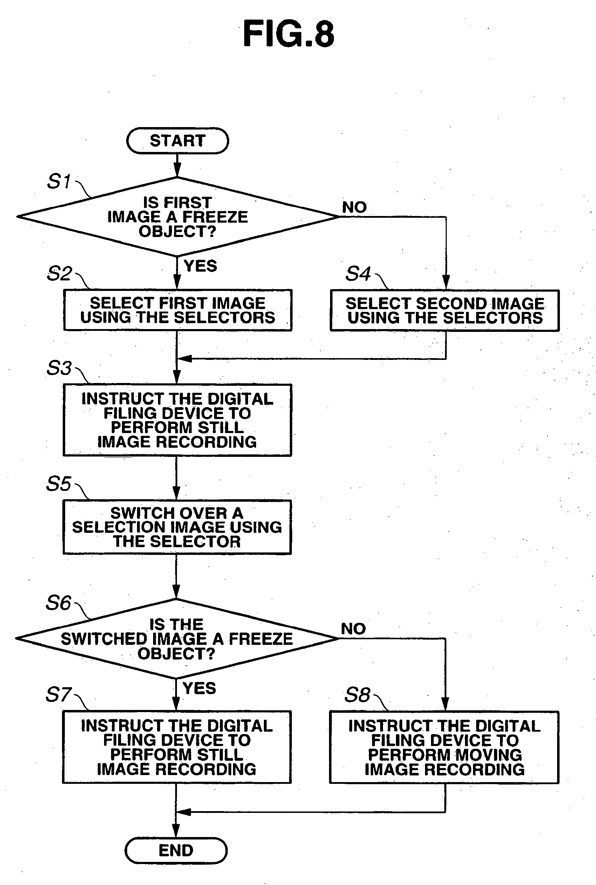

Also, upon the CPU 59 detecting operation of the release switch 29, as illustrated in Fig. 8, the CPU 59 determines in step S1 whether or not the first image (e.g., ordinary light image) is the freeze object image subjected to freeze processing by the freeze switch 28.

Subsequently, in the event that the first image (e.g., ordinary light image) is a freeze object image, the CPU 59 controls the selectors 58 and 62 to select the output of the freeze memory 61 of the freeze memory unit 54 in step S2, and outputs the freeze image of the first image (e.g., ordinary light image) to the digital filing device 6 in step S3.

In the event that the first image (e.g., ordinary light image) is not a freeze object image, the CPU 59 controls the selectors 58 and 62 to select the output of the freeze memory 61 of the freeze memory unit 55 in step S4, and outputs the freeze image of the second image to the digital filing device 6 in step S3.

Next, the CPU 59 controls the selector 58 to switch over an image in step S5, and determines in step S6 whether or not the switched image is a freeze object image.

In the event that the switched image is a freeze object image, the CPU 59 selects the output of the freeze memory 61, and outputs the freeze image to the digital filing device 6 in step S7. In the event that the switched image is not a freeze object image, the CPU 59 outputs the switched image as it is, i.e., as a moving image to the digital filing device 6 in step S8.

Thus, the present embodiment provides an advantage wherein the images of the two screen display on the same monitor having a different observation mode can be converted into the images suitable for observation.

### Second Embodiment

A second embodiment is almost the same as the first embodiment, so different points alone will be described, the same configurations are appended with the same reference numerals, and description thereof will be omitted.

As illustrated in Fig. 9, the present embodiment provides a contact observation optical system including a contact objective lens 81 and a contact observation CCD 82 instead of the fluorescent CCD 26, with the electronic endoscope 3. This contact observation optical system has been disclosed, for example, in Japanese Unexamined Patent Application Publication No. 2004-166913 (Fig. 4 in this patent document) and so forth, so is a known system, and accordingly, description thereof will be omitted.

Also, the video processor 5 includes a contact observation image video circuit 83 for subjecting the image capturing signal from the contact observation CCD 82 to signal processing to generate a contact observation image, and outputting this to the image synthetic circuit 57 via the freeze memory unit 84.

Also, the electronic endoscope 3 provides a second freeze switch 28a instead of the freeze selection switch 27, and as illustrated in Figs. 10A and 10B, the freeze switch 28 gives instructions for generating the still image of an ordinary light image, and the second freeze switch 28a gives instructions for generating the still image of a contact observation image.

Note that the freeze memory unit 84 has the same configuration as the freeze memory units 54 and 55 (see Fig. 3). The other configurations are the same as those in the first embodiment.

With the processing of the CPU 59 for performing image recording to the digital filing device 6 according to the present embodiment, upon detecting operation of the release switch 29, the CPU 59 controls the selectors 62 and 58 as illustrated in Fig. 11 to select the still image data of the first image stored in the freeze memory 61 of the freeze memory unit 54 in step S11, and outputs the still image of the selected first image to the digital filing device 6 in step S12.

Next, the CPU 59 controls the selectors 62 and 58 to select the still image data of the second image stored in the freeze memory 61 of the freeze memory unit 84 in step S13, and outputs the still image of the selected second image to the digital filing device 6 in step S14.

This processing enables the digital filing device 6 to record the still image having high resolution before being subjected to processing by the image synthetic circuit 57 in a state in which attribution information made up of information of the observation mode and so forth is appended. Accordingly, in the event that only the images having a certain observation mode are searched and shown by a list by recoding the first image and the second image individually, effective processing can be performed without referencing unnecessary images.

Note that with the above embodiments, the observation mode is not restricted to the above modes (e.g., ordinary light observation, narrowband light observation, and fluorescent observation), so an infrared light observation mode or ultraviolet light observation mode may be employed. Also, it is needless to say that the above embodiments can be applied to multi-screen display, which is not two-screen display, having more screens than two screens.

With the present invention, it is apparent that embodiments different in a wide range can be formed on the basis of this invention without departing from the scope of the invention as defined in the appended claims.

## Claims

1. An image processing device comprising:
a plurality of observation mode image processing means (51, 53, 83) for performing image processing to simultaneously display observation images of a subject in a plurality of observation modes;
freeze image selecting means (27) for setting one or more of the observation images in the plurality of observation modes to be an object image or object images of freeze processing;
freeze instructing means (28) different from the image selecting means (27) for giving an instruction to perform the freeze processing of the object image or the object images selected by the freeze image selecting means (27);
processing control means (59) for controlling the plurality of observation mode image processing means (51, 53, 83) so as to perform the freeze processing on the one or more of the observation images among the simultaneously displayed observation images in the plurality of observation modes, based on the instruction to perform the freeze processing by the freeze instructing means (28); and
image synthesizing means (57) for synthesizing the observation images generated by the observation mode image processing means (51, 53, 83) to display only the one or more observation images instructed to be frozen as a still image or still images, among the simultaneously displayed observation images in the plurality of observation modes, when the instruction to perform the freeze processing is given.

2. The image processing device according to Claim 1, further comprises image recording instruction means for instructing an image recording device (6) to record the observation images in the plurality of observation modes generated by the observation mode image processing means (51, 53,83).

3. The image processing device according to Claim 1 or Claim 2, wherein the observation mode image processing means (51, 53, 83) include freeze image storing means (54, 55, 84) for storing the freeze image of the observation image.

4. The image processing device according to Claim 3, wherein the freeze image storing means (54, 55, 84) is adapted to store the freeze image of the observation image to which attribute information including information showing the observation mode of the freeze image of the observation image has been added.

## Patentansprüche

1. Bildverarbeitungseinrichtung, die umfasst:
eine Mehrzahl von Beobachtungsmodusbildverarbeitungseininrichtungen (51, 53, 83) zum Durchführen einer Bildverarbeitung zum gleichzeitigen Darstellen von Beobachtungsbildern eines Subjekts in einer Mehrzahl von Beobachtungsmoden;
eine Standbildauswahleinrichtung (27) zum Festlegen eines oder mehrerer der Beobachtungsbilder in der Mehrzahl von Beobachtungsmoden als ein Objektbild oder Objektbilder einer Einfrierverarbeitung;
eine sich von der Bildauswahleinrichtung (27) unterscheidende Einfrierbefehlseinrichtung (28) zum Ausgeben eines Befehls zum Durchführen der Einfrierverarbeitung des Objektbilds oder der Objektbilder, das/die von der Standbildauswahleinrichtung (27) ausgewählt wurde/wurden;
eine Verarbeitungssteuerungseinrichtung (59) zur Steuerung der Mehrzahl von Beobachtungsmodusbildverarbeitungseininrichtungen (51, 53, 83), um basierend auf dem Befehl zum Durchführen der Einfrierverarbeitung von der Einfrierbefehlseinrichtung (28) die Einfrierverarbeitung an dem einen Beobachtungsbild oder den mehreren Beobachtungsbildern unter den gleichzeitig dargestellten Beobachtungsbildern in der Mehrzahl von Beobachtungsmoden durchzuführen; und
eine Bildsynthetisierungseinrichtung (57) zum Synthetisieren der durch die Beobachtungsmodusbildverarbeitungseininrichtungen (51, 53, 83) erzeugten Beobachtungsbilder, um nur das eine Beobachtungsbild oder die mehreren Beobachtungsbilder unter den gleichzeitig dargestellten Beobachtungsbildern in der Mehrzahl von Beobachtungsmoden darzustellen, für das/die der Befehl gegeben wurde, es/sie als ein Standbild oder Standbilder einzufrieren, wenn der Befehl zur Durchführung der Einfrierverarbeitung gegeben wird.

2. Bildverarbeitungseinrichtung gemäß Anspruch 1, das ferner eine Bildaufnahmebefehlseinrichtung zum Instruieren eines Bildaufnahmegeräts (6) umfasst, die Beobachtungsbilder in der Mehrzahl von Beobachtungsmoden, die durch die Beobachtungsmodusbildverarbeitungseininrichtungen (51, 53, 83) erzeugt werden, aufzunehmen.

3. Bildverarbeitungseinrichtung gemäß Anspruch 1 oder Anspruch 2, wobei die Beobachtungsmodusbildverarbeitungseininrichtungen (51, 53, 83) eine Standbildspeichereinrichtung (54, 55, 84) zum Speichern des Standbilds des Beobachtungsbilds umfasst.

4. Bildverarbeitungseinrichtung gemäß Anspruch 3, wobei die Standbildspeichereinrichtung (54, 55, 84) dazu eingerichtet ist, das Standbild des Beobachtungsbilds zu speichern, zu dem eine Attributinformation einschließlich einer Information, die den Beobachtungsmodus des Standbilds des Beobachtungsbilds zeigt, hinzugefügt wurde.

## Revendications

1. Dispositif de traitement d'images comprenant :
une pluralité de moyens de traitement (51, 53, 83) d'images en mode d'observation pour exécuter un traitement d'images pour afficher simultanément des images d'observation d'un sujet dans une pluralité de modes d'observation ;
un moyen de sélection (27) d'images à geler pour définir une ou plusieurs des images d'observation dans la pluralité de modes d'observation comme étant une image objet ou des images objets d'un traitement de gel ;
un moyen d'instruction (28) de gel différent du moyen de sélection (27) d'images pour donner une instruction d'exécution du traitement de gel de l'image objet ou des images objets sélectionnée(s) par le moyen de sélection (27) d'images à geler ;
un moyen de commande (59) de traitement pour commander la pluralité de moyens de traitement (51, 53, 83) d'images en mode d'observation de façon à exécuter le traitement de gel sur la ou les plusieurs des images d'observation parmi les images d'observation affichées simultanément dans la pluralité de modes d'observation, sur la base de l'instruction d'exécution du traitement de gel par le moyen d'instruction (28) de gel ; et
un moyen de synthèse (57) d'images pour synthétiser les images d'observation générées par les moyens de traitement (51, 53, 83) d'images en mode d'observation pour n'afficher que la ou les images d'observation indiquées comme devant être gelées comme une image fixe ou des images fixes, parmi les images d'observation affichées simultanément dans la pluralité de modes d'observation, lorsque l'instruction d'exécution du traitement de gel est donnée.

2. Dispositif de traitement d'images selon la revendication 1, comprenant en outre un moyen d'instruction d'enregistrement d'images pour indiquer à un dispositif (6) d'enregistrement d'images d'enregistrer les images d'observation dans la pluralité de modes d'observation générées par les moyens de traitement (51, 53, 83) d'images en mode d'observation.

3. Dispositif de traitement d'images selon la revendication 1 ou la revendication 2, dans lequel les moyens de traitement (51, 53, 83) d'images en mode d'observation incluent des moyens de stockage (54, 55, 84) d'image à geler pour stocker l'image à geler de l'image d'observation.

4. Dispositif de traitement d'images selon la revendication 3, dans lequel les moyens de stockage (54, 55, 84) d'image à geler sont adaptés à stocker l'image à geler de l'image d'observation à laquelle des informations d'attributs incluant une information montrant le mode d'observation de l'image à geler de l'image d'observation ont été ajoutées.
